# EUROPÄISCHE PATENTSCHRIFT

(11) **EP 0 602 522 B1**
(45) Veröffentlichungstag und Bekanntmachung des Hinweises auf die Patenterteilung: **29.05.1996**
(21) Anmeldenummer: 93119781.8
(22) Anmeldetag: 08.12.1993
(51) Int. Cl.: C07C 317/46, C07C 317/48, C07C 323/65, C07C 317/44, A61K 31/155

(54) **Substituierte Benzoylguanidine, Verfahren zu ihrer Herstellung, ihre Verwendung als Antiarrhythmika**
Substituted Benzoylguanidines, method for their preparation, their use as antiarrhythmic agents
Benzoylguanidines substituées, leur procédé de préparation, leur utilisation comme agents antiarrhythmiques

(30) Priorität: 15.12.1992 DE 4242192
(43) Veröffentlichungstag der Anmeldung: 22.06.1994
(73) Patentinhaber: HOECHST AKTIENGESELLSCHAFT, D-65929 Frankfurt am Main (DE)
(72) Erfinder: Weichert, Andreas, Dr., D-60529 Frankfurt/Main (DE); Lang, Hans-Jochen, Dr., D-65719 Hofheim/Taunus (DE); Kleemann, Heinz-Werner, Dr., D-61350 Bad Homburg (DE); Scholz, Wolfgang, Dr., D-65760 Eschborn (DE); Albus, Udo, Dr., D-61197 Florstadt (DE)

(56) Entgegenhaltungen:
- EP-A- 0 416 499

## Beschreibung

Die Erfindung betrifft Benzoylguanidine der Formel I 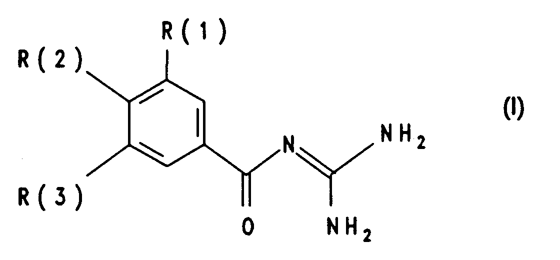
worin bedeuten:
- R(1): Wasserstoff, F, Cl, Br, I, -NO₂, -C≡N, -CF₃, R(4)-SOₘ oder R(5)R(6)N-SO₂-,
- m: Null, 1 oder 2,
- R(4) und R(5): (C₁-C₈)-Alkyl, (C₃-C₆)-Alkenyl, -CₙH₂ₙ-R(7), CF₃,
- n: Null, 1, 2, 3 oder 4,
- R(7): (C₃-C₇)-Cycloalkyl, Phenyl,
welches nicht substituiert ist oder substituiert mit 1 - 3 Substituenten aus der Gruppe
F, Cl, CF₃, Methyl, Methoxy oder NR(8)R(9) mit R(8) und R(9) gleich H oder C₁-C₄-Alkyl,
wobei R(5) auch in der Bedeutung von H steht,
- R(6): H, (C₁-C₄)-Alkyl,
wobei R(5) und R(6) gemeinsam 4 oder 5 Methylengruppen sein können, von denen eine CH₂-Gruppe durch Sauerstoff, S, NH, N-CH₃ oder N-Benzyl ersetzt sein kann,
- R(2): = -SR(10), -OR(10), -NHR(10), -NR(10)R(11), -CHR(10)R(12), 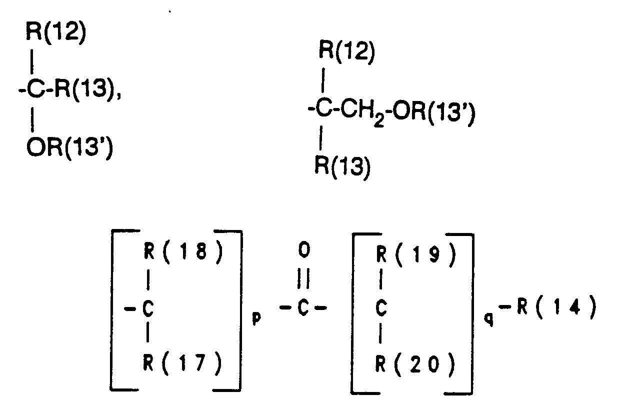
- R(10), R(11): gleich oder verschieden
-[CHR(16)]ₛ-(CH₂)ₚ-(CHOH)_{q}-(CH₂)ᵣ-(CH OH)ₜ-R(21) -(CH₂)ₚ-O-(CH₂-CH₂O)_{q}-R(21),
- R(21): Wasserstoff, Methyl,
- p, q, r: gleich oder verschieden
Null, 1, 2, 3 oder 4,
- s: Null, 1,
- t: 1, 2, 3 oder 4,
- R(12), R(13): gleich oder verschieden
Wasserstoff, (C₁-C₆)-Alkyl oder zusammen mit dem sie tragenden Kohlenstoff-Atom ein (C₃-C₈)-Cycloalkyl,
- R(13'): Wasserstoff, (C₁-C₄)-Alkyl,
- R(14): H, (C₁-C₆)-Alkyl, (C₃-C₈)-Cycloalkyl, -CₐH₂ₐ-R(15),
- a: Null, 1, 2, 3 oder 4,
- R(15): Phenyl,
das unsubstituiert oder substituiert ist mit 1 - 3 Substituenten aus der Gruppe F, Cl, CF₃, Methyl, Methoxy oder NR(8)R(9) mit R(8) und R(9) gleich H oder (C₁-C₄)-Alkyl,
(C₁-C₉)-Heteroaryl,
das unsubstituiert oder wie Phenyl substituiert ist,
(C₁-C₆)-Alkyl, das unsubstituiert oder mit 1-3 OH substituiert ist,
- R(16), R(17), R(18), R(19) und R(20): Wasserstoff, (C₁-C₃)-Alkyl,
- R(3): wie R(1) definiert,
oder
(C₁-C₆)-Alkyl, -X-R(22),
- X: Sauerstoff, S, NR(16),
R(16) H, (C₁-C₃)-Alkyl,
wobei R(22) und R(16) auch gemeinsam 4 oder 5 Methylengruppen sein können und eine CH₂-Gruppe durch Sauerstoff, S, NH, N-CH₃ oder N-Benzyl ersetzt sein kann, R(22) wie R(14) definiert ist;
sowie deren pharmazeutisch verträgliche Salze.

Bevorzugt sind Verbindungen der Formel I, in der bedeuten:
- R(1): Wasserstoff, F, Cl, -C≡N, -CF₃, R(4)-SOₘ oder R(5)R(6)N-SO₂-,
- m: Null, 1, 2,
- R(4) und R(5): (C₁-C₈)-Alkyl, (C₃-C₄)-Alkenyl, -CₙH₂ₙ-R(7), -CF₃,
- n: Null, 1,
- R(7): (C₃-C₆)-Cycloalkyl, Phenyl,
welches unsubstituiert ist oder substituiert mit 1 - 3 Substituenten aus der Gruppe F, Cl, CF₃, Methyl, Methoxy oder NR(8)R(9) mit R(8) und R(9) gleich H oder Methyl,
wobei R(5) auch in der Bedeutung von H steht,
- R(6): gleich H oder Methyl,
- R(3): Wasserstoff, Methyl, Cyano, -CF₃, F, Cl
und die übrigen Reste wie oben definiert sind,
sowie deren pharmazeutisch verträgliche Salze.

Besonders bevorzugt sind Verbindungen I, in denen bedeuten:
- R(1): F, Cl, -C≡N, -CF₃, R(4)-SOₘ oder R(5)R(6)N-SO₂-,
- m: Null, 1, 2,
- R(4): Methyl, CF₃,
- R(5), R(6): unabhängig voneinander
H oder Methyl;
- R(2): = -SR(10), -OR(10), NHR(10), -NR(10)R(11), -CHR(10)R(12), 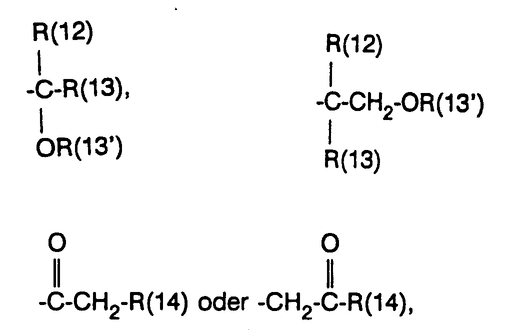
- R(10), R(11): gleich oder verschieden
-CH₂-(CHOH)_{q}-CHOH-CHOH-CHOH-CH₂OH, -CH₂-CHOH-CH₂OH, -[CHR(16)]ₛ-CH₂-CHOH-R(21) oder -(CH₂)ₚ-O-(CH₂-CH₂-O)_{q}-CH₃,
- p: Null, 1, 2,
- q: Null, 1, 2,
- s: Null, 1,
- R(21): Wasserstoff, Methyl,
- R(12), R(13): - gleich oder verschieden -
Wasserstoff, Methyl, oder zusammen mit dem sie tragenden Kohlenstoffatom ein (C₃-C₈)-Cycloalkyl,
- R(13'): Wasserstoff, Methyl,
- R(14): H, (C₁-C₆)-Alkyl, (C₃-C₈)-Cycloalkyl, -CₐH₂ₐ-R(15), a 0, 1,
- R(15): Phenyl,
das unsubstituiert oder substituiert ist mit 1 - 2 Resten aus der Reihe F, Cl, CF₃, -CH₃,
oder Heteroaryl aus der Reihe Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl,
die unsubstituiert oder mit einem Rest aus der Reihe F, Cl, CF₃, -CH₃ substituiert sind,
(C₁-C₄)-Alkyl, das mit einer OH substituiert ist;
- R(16): Wasserstoff, Methyl,
- R(3): Methyl, Cyano, Trifluormethyl, F, Cl, Wasserstoff,
sowie deren pharmazeutisch verträgliche Salze.

Ganz besonders bevorzugt sind Verbindungen mit
- R(15): Phenyl,
das unsubstituiert oder substituiert ist mit 1 - 2 Resten aus der Reihe F, Cl, CF₃,
Imidazolyl, Tetrazolyl, Pyridinyl, Pyrimidinyl,
die unsubstituiert oder mit einem Rest aus der Reihe F, Cl, CF₃, CH₃ substituiert sind,
Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind.

Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(22) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man
Verbindungen der Formel II 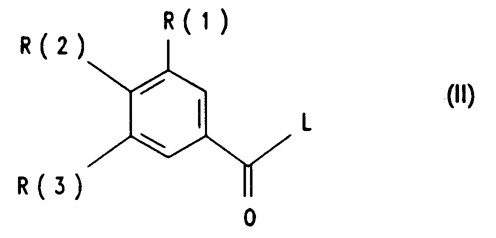
mit Guanidin umsetzt, worin R(1) bis R(3) die angegebene Bedeutung besitzen und L für eine leicht nucleophil substituierte leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L= OH) beispielsweise mit Thionylchlorid herstellen kann.

Neben den Carbonsäurechloriden der Formel II (L=Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCl in Methanol, die Imidazolide der Formel II durch Behandeln mit Carbonyldiimidazol [L= 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluor borat ("TOTU")[Weiss und Krommer, Chemiker Zeitung 98, 817 (1974)]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L=OMe) mit Guanidin Methanol, Isopropanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L=Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 4-(bzw. 5-)Halogen-3-chlor-sulfonylbenzoesäuren mit Ammoniak oder Aminen in 3-Aminosulfonyl-4-(bzw. 5-)halogen-benzoesäuren bzw. mit einem schwachen Reduktionsmittel wie Natriumbisulfit und anschließender Alkylierung in 3-Alkylsulfonyl-4-(bzw. 5-)halogen-benzoesäuren überführt und die erhaltenen Benzoesäuren nach einer der oben beschriebenen Verfahrensvarianten zu erfindungsgemäßen Verbindungen I umgesetzt werden.

Die Einführung einiger Substituenten in 4- und 5-Stellung gelingt durch literaturbekannte Methoden des Palladium-vermittelten cross-couplings von Arylhalogeniden mit beispielsweise Organostannanen, Organoboronsäuren oder Organoboranen oder Organokupfer- bzw. zink-verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 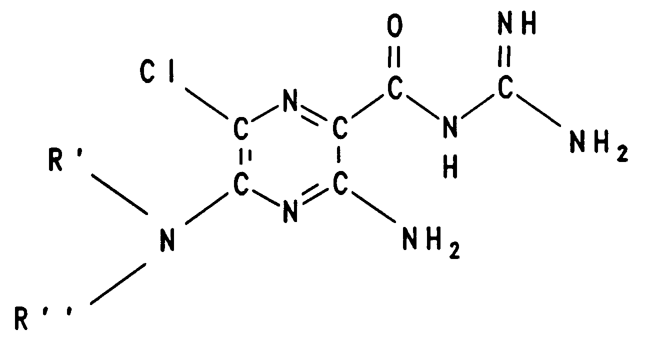
Amilorid: R',R'' = H
Dimethylamilorid: R',R'' = CH₃
Ethylisopropylamilorid: R' = C₂H₅, R'' = CH(CH₃)₂
Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden 3,5-substituierte Benzoylguanidine vorgeschlagen, in welchen aber die Substituenten R(2) und R(3) nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na+/H+ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers. Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- NBS: N-Bromsuccinimid
- AIBN: α,α-Azo-bis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent

### Experimenteller Teil

### Allgemeine Vorschrift zur Herstellung von Benzoyl-guanidinen (I)

### Variante A: aus Benzoesäuren (II, L=OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L=O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1

### 4-(1'-Hydroxy-1'-methyl)-ethyl-3-methylsulfonyl-benzoylguanidin-hydrochlorid:

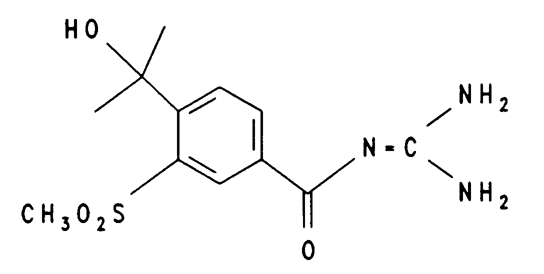

### Syntheseweg:

a) Oxidation von 4-Ethyl-3-methylsulfonyl-benzoesäuremethylester zu 4-Acetyl-3-methylsulfonyl-benzoesäuremethylester in Eisessig mit KMnO₄/ Benzyltriethylammoniumpermanganat (2 : 1 eq) bei RT für zwei Tage, nach Zusatz von Wasser und NaHCO₃ mit EE extrahieren und org. Phase trocknen (MgSO₄), Verreiben in Ethanol, farblose Kristalle abfiltrieren, mp 111°C
b) 4-(1'-Hydroxy-1'-methyl)-ethyl-3-methylsulfonyl-benzoesäuremethylester aus a) in Methylenchlorid zu einer Lösung aus 2 eq Dimethylzink und 2 eq TiCl₄ bei -20°C gegeben und während 3 Stunden auf RT erwärmen lassen, die Mischung in Wasser gießen, mit CH₂Cl₂ ausschütteln, trocknen (MgSO₄) und Lösungsmittel im Vakuum entfernen. Farblose Kristalle, mp 133 bis 135°C
c) 4-(1'-Hydroxy-1'-methyl)-ethyl-3-methylsulfonyl-benzoylguanidin-hydrochlorid aus b) nach der allg. Vorschrift B (siehe oben). Farblose Kristalle, mp 219 bis 220°C

### Beispiel 2

### 4-(2-Methoxy-ethoxy)methoxy-3-methylsulfonyl-benzoylguanidin, Methansulfonsäuresalz

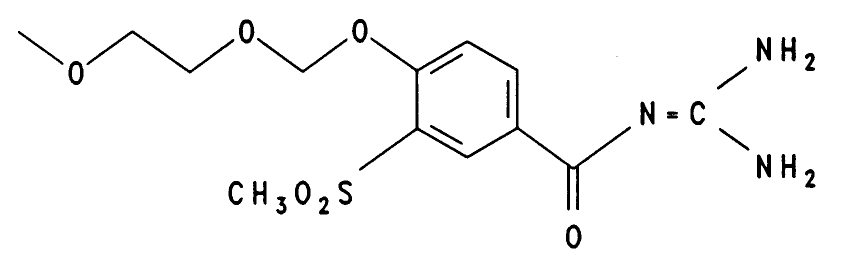

### Syntheseweg:

a) 4-Hydroxy-3-methylsulfonyl-benzoesäure-methylester
   6 mmol 4-Chlor-3-methylsulfonyl-benzoesäure-methylester und 6 mmol H₂O werden in 30 ml Tetramethylharnstoff gelöst, dann werden 18 mmol K₂CO₃ addiert, und es wird für 2 h bei 130°C gerührt. Das abgekühlte Reaktionsgemisch wird in 100 ml gesättigte wäßrige NaHCO₃-Lösung gegossen und 5 x mit je 100 ml EE extrahiert. Über Na₂SO₄ wird getrocknet, das Lösungsmittel im Vakuum entfernt und der Rückstand an Kieselgel mit EE chromatographiert.
   R_{f} (EE) = 0,36 MS(DCl): 231 (M+1)
b) 4-(2-Methoxy-ethoxy)methoxy-3-methylsulfonyl-benzoesäure-methylester
   2,2 mmol des Phenols aus 2a) und 4,4 mmol Ethyldiisopropylamin werden in 10 ml CH₂Cl₂ gelöst, und dann werden bei RT 3,3 mmol (2-Methoxy-ethoxy)methylchlorid zugespritzt. 3 Tage wird bei RT gerührt, dann wird das Lösungsmittel im Vakuum entfernt, und der Rückstand wird in 100 ml EE aufgenommen. 3 x wird mit je 50 ml 0,3 M KH₂PO₄, dann 2 x mit je 50 ml Na₂CO₃ gewaschen. Über Na₂SO₄ wird getrocknet, das Lösungsmittel im Vakuum entfernt, und die Substanz wird ohne zusätzliche Reinigung weiter eingesetzt.
   R_{f} (EE) = 0,45 MS(DCl): 319 (M+1)
c) Die Titelverbindung des Beispiels 2 erhält man aus b) nach der allgemeinen Vorschrift B. Die freie Base wird in MeOH gelöst und mit einem Äquivalent Methansulfonsäure versetzt. Das Salz wird mit DIP ausgefällt und abgesaugt. Farblose Kristalle, mp 167°C MS(DCl): 346 (M+1)

### Beispiel 3

### 4-[2(R),3(R),4(R),5(R),6-Pentahydroxy-hexylamino]-3-methylsulfonyl-benzoylguanidin, Hydrochlorid

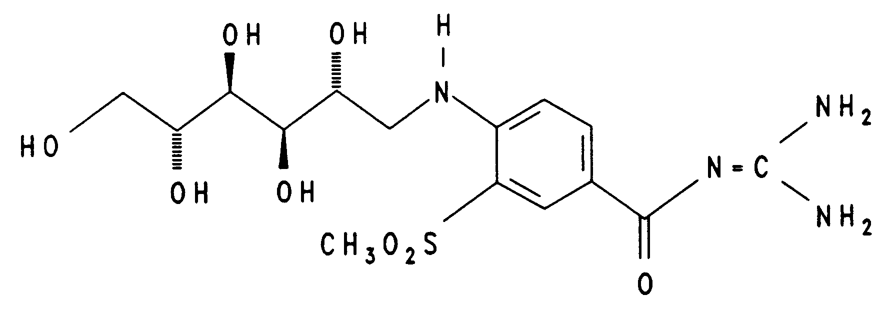

### Syntheseweg:

a) N-Benzhydryl-N-[2(R),3(R),4(R),4(R),6-Pentahydroxy-hexyl]-amin 2,3:5,6-Diacetonid
   24 mmol N-Benzhydrylmannofuranosylamin 2,3:5,6-Diacetonid (J. Med. Chem. 1992, 35, 559) werden in 150 ml THF gelöst und bei RT portionsweise mit 60 mmol LiAlH₄ versetzt. 2 h wird bei RT gerührt, anschließend auf 250 ml NaHCO₃-Lösung gegossen und 3 x mit je 200 ml EE extrahiert. Über Na₂SO₄ wird getrocknet, das Lösungsmittel im Vakuum entfernt und der Rückstand ohne zusätzliche Reinigung weiter eingesetzt. Farbloses Öl.
   R_{f} (DIP) = 0,33 MS(FAB): 428 (M+1)
b) [2(R),3(R),4(R),5(R),6-Pentahydroxy-hexyl]-amin 2,3:5,6-Diacetonid
   24 mmol der Verbindung aus 3a) werden in 200 ml MeOH gelöst und mit 240 mmol Ammoniumformiat sowie 2 g Pd/C versetzt und 4 h bei RT gerührt. Das Gemisch wird abfiltriert und das Lösungsmittel im Vakuum entfernt; anschließend wird in 100 ml EE/100 ml Na₂CO₃-Lösung aufgenommen. Danach wird noch 2 x mit je 200 ml EE extrahiert, über Na₂SO₄ getrocknet, und das Lösungsmittel wird im Vakuum entfernt. Der Rückstand wird an Kieselgel mit EE/MeOH 1:1 chromatographiert. Farbloses Öl.
   R_{f} (EE/MeOH 1:1) = 0,2 MS(DCl): 262 (M+1)
c) 4-[2(R),3(R),4(R),5(R),6-Pentahydroxy-hexylamino]-3-methylsulfonyl-benzoesäure 2,3:5,6-Diacetonid
   2,3 mmol des Amins aus 3b), 2,3 mmol 4-Fluor-3-methylsulfonyl-benzoesäure sowie 4,6 mmol Diisopropylethylamin werden in 10 ml Tetramethylharnstoff gelöst und 3 h bei 120°C gerührt. Anschließend wird das Lösungsmittel im Vakuum entfernt, und der Rückstand wird an Kieselgel mit EE/MeOH 10:1 chromatographiert. Man erhält ein hellbraunes Öl.
   R_{f} (EE/MeOH 5:1) = 0,5 MS(FAB): 460 (M+1)
d) 4-[2(R),3(R),4(R),5(R),6-Pentahydroxy-hexylamino]-3-methylsulfonyl-benzoylguanidin 2,3:5,6-Diacetonid
   2 mmol der Benzoesäure 2c) werden nach der allgemeinen Vorschrift A umgesetzt und an Kieselgel mit EE/MeOH 10:1 chromatographiert.
   Farbloses Öl. R_{f} (EE/MeOH 10:1) = 0,14 MS(FAB): 501 (M+1)
e) Zur Synthese der Titelverbindung des Beispiels 3 werden 0,6 mmol Diacetonid 3d) zusammen mit 2,4 mmol p-Toluolsulfonsäure in 10 ml MeOH gelöst und 2 h bei RT gerührt. Über basischen Ionenaustauscher wird filtriert und das Lösungsmittel im Vakuum entfernt. Farbloses, hygroskopisches Öl.
   R_{f} (Aceton/H₂O 10:1) = 0,09 MS(FAB): 421 (M+1)
   Zur Aufbewahrung wurde das Produkt in das Hydrochlorid überführt. mp 188°C

### Beispiel 4

### 4-[2(S)-hydroxy-propylamino]-3-methylsulfonyl-benzoylguanidin Hydrochlorid

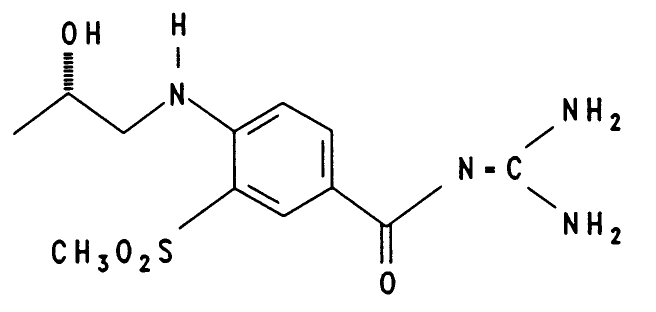

### Syntheseweg:

a) 4-[2(S)-Hydroxy-propylamino]-3-methylsulfonyl-benzoesäure
   10 mmol 2(S)-Hydroxy-propylamin werden analog 3c) mit 4-Fluor-3-methylsulfonyl-benzoesäure umgesetzt. Bräunliche Kristalle.
   mp 158 bis 160°C MS(DCl): 274 (M+1)
b) 4-[2(S)-Hydroxy-propylamino]-3-methylsulfonyl-benzoesäure-methylester
   7 mmol der Benzoesäure 4a) werden zusammen mit 14 mmol SOCl₂ in 30 ml MeOH gelöst und 3 h unter Rückfluß gekocht. Anschließend wird das Lösungsmittel im Vakuum entfernt, in 100 ml EE aufgenommen und 3 x mit je 50 ml Na₂CO₃-Lösung gewaschen. Über Na₂SO₄ wird getrocknet, das Lösungsmittel im Vakuum entfernt, und dann wird der Rückstand aus EE/HEP umkristallisiert.
   mp 95°C
   R_{f} (MTB) = 0,30 MS(DCl): 288
c) Zur Synthese der Titelverbindung des Beispiels 4 werden 5 mmol des Methylester 4b) nach der allgemeinen Vorschrift B umgesetzt und an Kieselgel mit EE/MeOH 5:1 chromatographiert.
   mp 136 bis 140°C R_{f} (EE/MeOH 5 : 1) = 0,14
   Umsetzung zum Hydrochlorid ergab farblose Kristalle
   mp 204°C MS(DCl): 315 (M+1)
Die Titelverbindung des Beispiels 5 wurde analog Beispiel 4 synthetisiert:

### Beispiel 5

### 4-[2(R)-Hydroxy-propylamino]-3-methylsulfonyl-benzoylguanidin Hydrochlorid

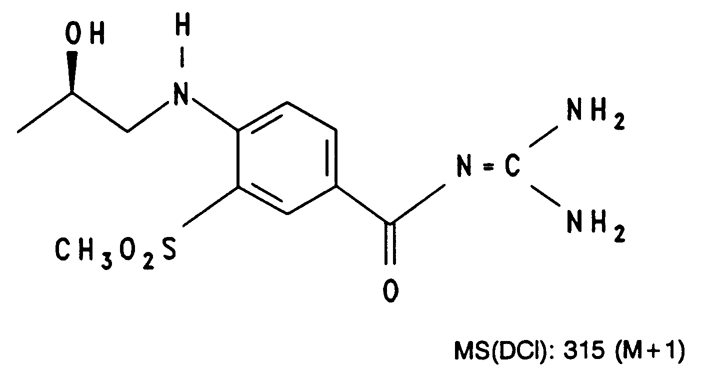

mp 203°C MS(DCl): 315 (M+1)

### Beispiel 6

### 3-Methylsulfonyl-4-[2(R,S),3-dihydroxypropyl]thio-benzoylguanidin

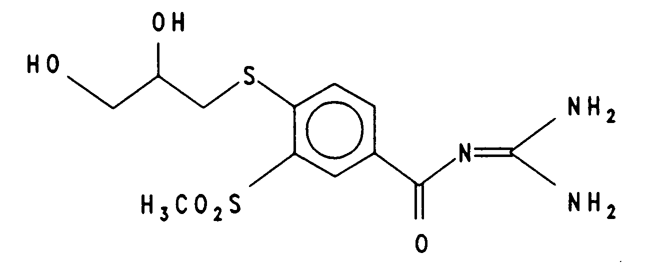

a) 3-Methylsulfonyl-4-[2(R,S),3-dihydroxypropyl]thio-benzoesäure-methylester
   20 mmol 4-Chlor-3-methylsulfonyl-benzoesäure-methylester, 20 mmol 1-Thioglycerin sowie 60 mmol K₂CO₃ (wasserfrei) werden in 70 ml Tetramethylharnstoff bei RT 24 h gerührt. Das Reaktionsprodukt wird in 30 ml Na₂CO₃ gegossen und 3 x mit 300 ml EE extrahiert. Über Na₂SO₄ wird getrocknet und die Lösungsmittel im Vakuum entfernt. Chromatographie an Kieselgel mit EE liefert farblose Kristalle. mp = 136°C
   R_{f} (EE) = 0,23 MS (DCl): 321 (M+1)
b) 3-Methylsulfonyl-4-[2(R,S),3-dihydroxypropyl]thio-benzoylguanidin
   5 mmol Methylester a) und 25 mmol Guanidin werden in 40 ml THF (wasserfrei) 6 h unter Rückfluß erhitzt. Das Gemisch wird in 100 ml ges. Na₂CO₃-Lösung gegossen und 3 x mit 150 ml EE extrahiert. Über Na₂SO₄ wird getrocknet und das Lösungsmittel im Vakuum entfernt. Chromatographie an Kieselgel mit EE/MeOH 3 : 1 liefert die Titelverbindung des Beispiels 6 als farblosen Schaum.
   R_{f} (EE/MeOH 3 : 1) = 0,23 MS (DCl): 348 (M+1)

### Beispiel 7

### 3-Methylsulfonyl-4-[1'-oxo-2'-phenyl-ethyl]-benzoylguanidin-hydrochlorid Farblose Kristalle, Smp. 198°C

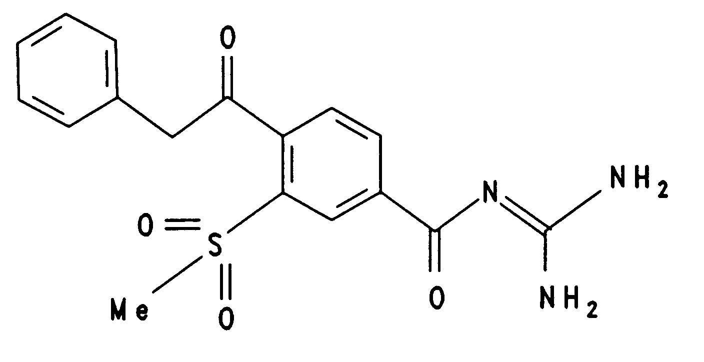

### Syntheseweg:

a) 3-Methylsulfonyl-4-[(2'-phenyl)-ethinyl]-benzoesäuremethylester aus 4-Bromo-3-methylsulfonyl-benzoesäuremethylester durch Stephans-Castro-coupling mit 2.5 Äquivalenten Phenylacetylen, Rühren bei RT für 24 h in Gegenwart von katal. (5 mol%) Bis-(Triphenylphosphin)-palladium(II)chlorid, 15 mol% Kupfer(I)iodid und 3 Äquivalenten n-Butylamin in THF, wäßrige Ammoniumchloridaufarbeitung, Extraktion mit Essigester und anschließende Säulenchromatographie an Kieselgel mit Essigester/Cyclohexan (3:7), farblose Kristalle, Smp. 138-39°C.
b) 3-Methylsulfonyl-4-[1'-oxo-2'-phenyl-ethyl]-benzoesäuremethylester aus a) durch Versetzen der essigsauren Lösung mit Quecksiber(II)acetat in Gegenwart von konz. Schwefelsäure, anschließendes Erhitzen auf 80°C für 3 h. Nach Filtration und Verdünnen mit Wasser wird mit Essigester extrahiert, mit ges. NaHCO₃-Lösung neutral gewaschen und der organische Extrakt säulenchromatographiert mit Cyclohexan/Essigester 1:1 als Laufmittelgemisch. Farblose Kristalle, Smp. 160-161°C.
c) 3-Methylsulfonyl-4-[1'-oxo-2'-phenyl-ethyl]-benzoesäure aus b) in Methanol durch Hydrolyse mit 1N NaOH bei Raumtemperatur. Farblose Kristalle, Smp. 229°C.
d) 3-Methylsulfonyl-4-[1'-oxo-2'-phenyl-ethyl]-benzoylguanidin-hydrochlorid aus c) analog Variante A.

### Beispiel 8

### 4-[2'-Cyclohexyl-1'-oxo-ethyl]-3-methylsulfonyl-benzoylguanidin-hydrochlorid Farblose Kristalle, Smp. 224-25°C

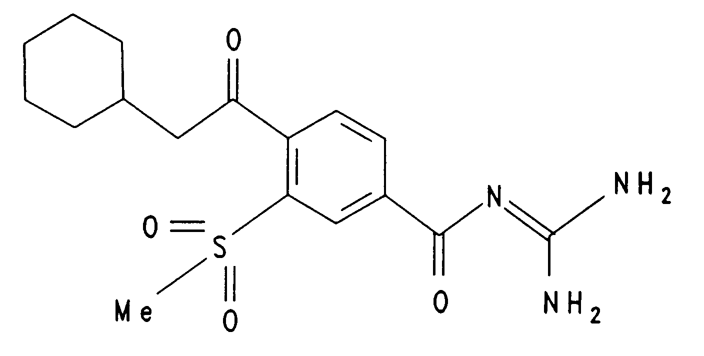

### Syntheseweg:

a) 4-[(2'-Cyclohexyl)-ethinyl]-3-methylsulfonyl-benzoesäuremethylester aus 4-Bromo-3-methylsulfonyl-benzoesäure-methylester durch Stephans-Castro-coupling wie für 7 a) beschrieben, Kopplungspartner Cyclohexylacetylen, farblose Kristalle, Smp. 81-82°C.
b) 3-Methylsulfonyl-4-[2'-cyclohexyl-1'-oxo-ethyl]-benzoesäuremethylester aus 8a) analog 7 b), farblose Kristalle, Smp. 130-31°C.
c) 3-Methylsulfonyl-4-[2'-cyclohexyl-1'-oxo-ethyl]-benzoesäure aus 8 b) analog 7c), farblose Kristalle, Smp. 174°C.
d) 4-[2'-Cyclohexyl-1'-oxo-ethyl]-3-methylsulfonyl-benzoylguanidin-hydrochlorid aus 8 c) nach Variante A.

### Beispiel 9

### 4-[1'-Hydroxy-2'-propyl]-3-methylsulfonyl-benzoylguanidin-hydrochlorid. Farblose Kristalle, Smp. 204-6°C

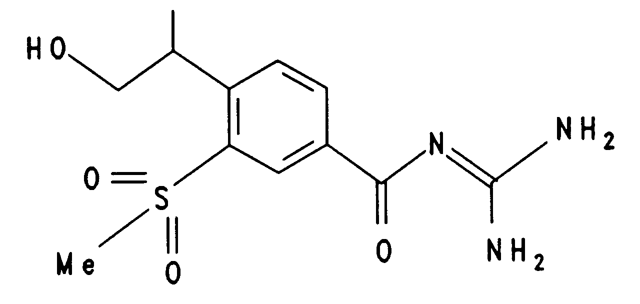

### Syntheseweg:

a) 4-[1'-Hydroxy-2'-propyl]-3-methylsulfonyl-benzoesäure aus 4-Isopropenyl-3-methylsulfonyl-benzoesäuremethylester (siehe Vorstufe 2) durch Hydroborierung mit 0.35 Äquivalenten Boran-Dimethylsulfidkomplex in THF unter Erhitzen am Rückfluß für 2 Tage. Nach Alkalisierung mit 2N NaOH wird mit 30%-H₂O₂-Lösung oxidiert. Wäßrige Aufarbeitung, Extraktion mit Essigester, Evaporierung und Verreiben mit Ether ergibt farblose Kristalle, Smp. 187-89°C
b) 4-[1'-Hydroxy-2'-propyl]-3-methylsulfonyl-benzoesäuremethylester aus 9 a) mit 1.2 Äquivalenten Methyliodid in Gegenwart von Kaliumcarbonat bei Erwärmen für 3 Stunden unter Rückfluß. Wäßrige Aufarbeitung, Säulenchromatograpie mit Cyclohexan/Essigester 1:1. Farblose Kristalle, Smp. 127-29°C.
c) 4-[1'-Hydroxy-2'-propyl]-3-methylsulfonyl-benzoylguanidin-hydrochlorid aus 9b) analog Variante B.

### Vorstufe 1

### Isopropenyl-boronsäure

90 mmol Isopropenylbromid und 99 mmol Mg werden in 50 ml Diethylether zur Grignard-Verbindung umgesetzt. Diese Suspension wird bei -60°C zu einer Lösung von 90 mmol Trimethylborat in 100 ml Diethylether langsam zugetropft. 1 h wird bei RT gerührt, das Solvens im Vakuum entfernt und 300 ml 4N NaOH-Lösung zugegeben. Anschließend wird das Mg(OH)₂ abgesaugt, mit 100 ml H₂O gewaschen und das Filtrat 2x mit je 100 ml DIP extrahiert. Dann wird die wäßrige Phase auf pH = 1 gestellt und 4x mit je 200 ml EE ertrahiert. Die EE-Phase wird über MgSO₄ getrocknet und das Solvens im Vakuum entfernt. Man erhält 2,0 g eines amorphen Feststoffs, der ohne Reinigung weiter umgesetzt wird.

### Vorstufe 2

### 4-Isoprenyl-3-methylsulfonyl-benzoesäuremethylester

23 mmol 4-Brom-3-methylsulfonyl-benzoesäuremethylester, 54 mmol Na₂CO₃, 2,9 mmol Triphenylphosphin und 1,5 mmol Pd(OAc)₂ werden in 200 ml Toluol und 15 ml H₂O 5 min. bei RT intensiv gerührt. Dann wird eine Lösung von 23 mmol Boronsäure nach Vorstufe 1 in 50 ml EtOH zugegeben und 1,5 h unter Rückfluß gekocht. Nach Phasentrennung wird die organische Phase 2x mit 50 ml NaCl-Lösung gewaschen und die wäßrige Phase 2x mit 100 ml EE extrahiert. Die vereinigten organischen Phasen werden über Na₂SO₄ getocknet, die Solventien im Vakuum entfernt und der Rückstand an Kieselgel mit DIP chromatographiert. Man erhält 1,4 g eines farblosen Öls.
R_{f} (DIP) = 0,46 MS(DCl): 255 (M+1)

### Beispiel 10

### 4-[1'-Methoxy-2'-propyl]-3-methylsulfonyl-benzoylguanidin-hydrochlorid. Farblose Kristalle, Smp. 190°C

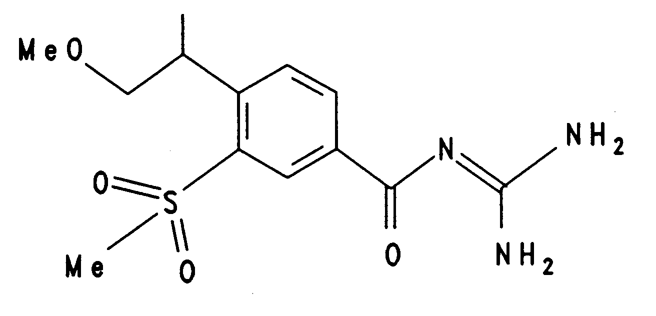

a) 4-[1'-Methoxy-2'propyl]-3-methylsulfonyl-benzoesäuremethylester aus 9 b mit Natriumhydrid in Gegenwart von 1.5 Äquivalenten Methyliodid durch Erwärmen auf 50°C für 4 Stunden in THF. Wäßrige Aufarbeitung, Säulenchromatographie Cyclohexan/Essigester 8:2. Farblose Kristalle, amorph.
b) 4-[1'-Methoxy-2'-propyl]-3-methylsulfonyl-benzoylguanidin-hydrochlorid aus 10 a) analog Variante B.

## Patentansprüche

1. Verbindung der Formel I 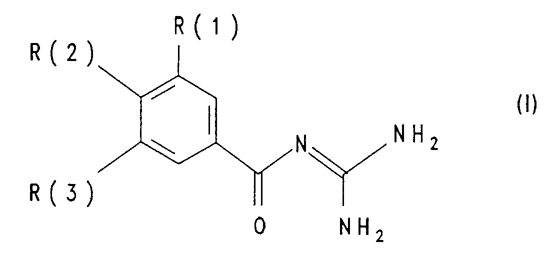 worin bedeuten:
R(1) Wasserstoff, F, Cl, Br, I, -NO₂, -C≡N, -CF₃, R(4)-SOₘ oder R(5)R(6)N-SO₂-,
m Null, 1 oder 2,
R(4) und R(5)
(C₁-C₈)-Alkyl, (C₃-C₆)-Alkenyl, -CₙH₂ₙ-R(7), CF₃,
n Null, 1, 2, 3 oder 4,
R(7) (C₃-C₇)-Cycloalkyl, Phenyl,
welches nicht substituiert ist oder substituiert mit 1 - 3 Substituenten aus der Gruppe F, Cl, CF₃, Methyl, Methoxy oder NR(8)R(9);
R(8) und R(9)
H oder C₁-C₄-Alkyl,
wobei R(5) auch in der Bedeutung von H steht,
R(6) H, (C₁-C₄)-Alkyl,
wobei R(5) und R(6) gemeinsam 4 oder 5 Methylengruppen sein können, von denen eine CH₂-Gruppe durch Sauerstoff, S, NH, N-CH₃ oder N-Benzyl ersetzt sein kann,
R(2) -SR(10), -OR(10), -NHR(10), -NR(10)R(11), -CHR(10)R(12), -CR(12)R(13)OR(13'), -CR(12)[CH₂O-R(13')]R(13), -[CR(17)R(18)]ₚ-CO-[CR(19)R(20)]_{q}-R(14),
R(10), R(11) gleich oder verschieden
-[CHR(16)]ₛ-(CH₂)ₚ-(CHOH)_{q}-(CH₂)ᵣ-(CHOH)ₜ-R(21), -(CH₂)ₚ-O-(CH₂-CH₂O)_{q}-R(21),
R(21) Wasserstoff, Methyl,
p, q, r gleich oder verschieden
Null, 1, 2, 3 oder 4,
s Null, 1,
t 1, 2, 3 oder 4,
R(12), R(13) gleich oder verschieden
Wasserstoff, (C₁-C₆)-Alkyl oder zusammen mit dem sie tragenden Kohlenstoff-Atom ein (C₃-C₈)-Cycloalkyl,
R(13') Wasserstoff, (C₁-C₄)-Alkyl,
R(14) H, (C₁-C₆)-Alkyl, (C₃-C₈)-Cycloalkyl, -CₐH₂ₐ-R(15),
a Null, 1, 2, 3 oder 4,
R(15) Phenyl,
das unsubstituiert oder substituiert ist mit 1 - 3 Substituenten aus der Gruppe F, Cl, CF₃, Methyl, Methoxy oder NR(8)R(9) mit R(8) und R(9) gleich H oder (C₁-C₄)-Alkyl,
(C₁-C₉)-Heteroaryl,
das unsubstituiert oder wie Phenyl substituiert ist,
(C₁-C₆)-Alkyl, das unsubstituiert oder mit 1-3 OH substituiert ist,
R(16), R(17), R(18), R(19) und R(20)
Wasserstoff, (C₁-C₃)-Alkyl,
R(3) wie R(1) definiert,
oder
(C₁-C₆)-Alkyl, -X-R(22),
X Sauerstoff, S, NR(16),
R(16) H, (C₁-C₃)-Alkyl,
wobei R(22) und R(16) auch gemeinsam 4 oder 5 Methylengruppen sein können und eine CH₂-Gruppe durch Sauerstoff, S, NH, N-CH₃ oder N-Benzyl ersetzt sein kann, R(22) wie R(14) definiert ist;
sowie deren pharmazeutisch verträgliche Salze.

2. Verbindung der Formel I nach Anspruch 1, in der bedeuten:
R(1) Wasserstoff, F, Cl, -C≡N, -CF₃, R(4)-SOₘ oder R(5)R(6)N-SO₂-,
m Null, 1, 2,
R(4) und R(5)
(C₁-C₈)-Alkyl, (C₃-C₄)-Alkenyl, -CₙH₂ₙ-R(7), -CF₃,
n Null, 1,
R(7) (C₃-C₆)-Cycloalkyl, Phenyl,
welches unsubstituiert ist oder substituiert mit 1 - 3 Substituenten aus der Gruppe F, Cl, CF₃, Methyl, Methoxy oder NR(8)R(9) mit R(8) und R(9) gleich H oder Methyl,
wobei R(5) auch in der Bedeutung von H steht,
R(6) H oder Methyl,
R(3) Wasserstoff, Methyl, Cyano, -CF₃, F, Cl,
und die übrigen Reste wie in Anspruch 1 definiert sind.

3. Verbindung der Formel I nach Anspruch 1, in der bedeuten:
R(1) F, Cl, -C≡N, -CF₃, R(4)-SOₘ oder R(5)R(6)N-SO₂-,
m Null, 1, 2,
R(4) Methyl, CF₃,
R(5), R(6) unabhängig voneinander
H oder Methyl;
R(2) -SR(10), -OR(10), NHR(10), -NR(10)R(11), -CHR(10)R(12), -CR(12)R(13)OR(13'), -CR(12)[CH₂-OR(13')]R(13), -CO-CH₂-R(14) oder -CH₂-CO-R(14),
R(10), R(11) gleich oder verschieden -CH₂-(CHOH)_{q}-CHOH-CHOH-CHOH-CH₂OH, -CH₂-CHOH-CH₂OH, -[CHR(16)]ₛ-CH₂-CHOH-R(21) oder -(CH₂)ₚ-O-(CH₂-CH₂-O)_{q}-CH₃,
p Null, 1, 2,
q Null, 1, 2,
s Null, 1,
R(21) Wasserstoff, Methyl,
R(12), R(13) - gleich oder verschieden - Wasserstoff, Methyl, oder zusammen mit dem sie tragenden Kohlenstoffatom ein (C₃-C₈)-Cycloalkyl,
R(13')
Wasserstoff, Methyl,
R(14)
H, (C₁-C₆)-Alkyl, (C₃-C₈)-Cycloalkyl, -CₐH₂ₐ-R(15),
a 0, 1,
R(15)
Phenyl,
das unsubstituiert oder substituiert ist mit 1 - 2 Resten aus der Reihe F, Cl, CF₃, -CH₃,
oder Heteroaryl aus der Reihe Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl,
die unsubstituiert oder mit einem Rest aus der Reihe F, Cl, CF₃, -CH₃ substituiert sind,
(C₁-C₄)-Alkyl, das mit einer OH substituiert ist;
R(16)
Wasserstoff, Methyl,
R(3) Methyl, Cyano, Trifluormethyl, F, Cl, Wasserstoff.

4. Verbindung der Formel I nach Anspruch 1, dadurch gekennzeichnet, daß bedeuten:
R(15) Phenyl,
das unsubstituiert oder substituiert ist mit 1 - 2 Resten aus der Reihe F, Cl, CF₃,
Imidazolyl, Tetrazolyl, Pyridinyl, Pyrimidinyl,
die unsubstituiert oder mit einem Rest aus der Reihe F, Cl, CF₃, CH₃ substituiert sind.

5. Verfahren zum Herstellen einer Verbindung I nach Anspruch 1, dadurch gekennzeichnet, daß man eine Verbindung der Formel II 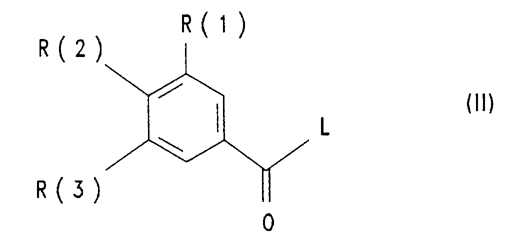 mit Guanidin umsetzt, worin R(1) bis R(3) die angegebene Bedeutung besitzen und L für eine leicht nucleophil substituierte leaving group steht.

6. Verwendung einer Verbindung I nach Anspruch 1 zur Herstellung eines Medikaments zur Behandlung von Arrhythmien.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Heilmittel, enthaltend eine wirksame Menge einer Verbindung I nach einem oder mehreren der Ansprüche 1 bis 4.

## Claims

1. A compound of the formula I 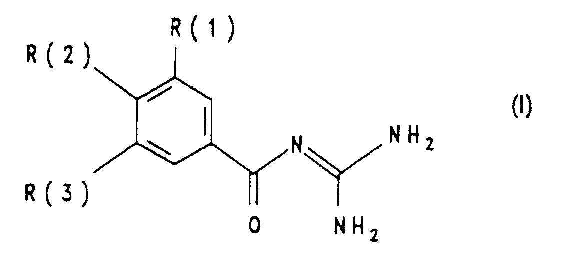 in which:
R(1) is hydrogen, F, Cl, Br, I, -NO₂, -C≡N, -CF₃, R(4)-SOₘ or R(5)R(6)N-SO₂-,
m is zero, 1 or 2,
R(4) and R(5)
are (C₁-C₈)-alkyl, (C₃-C₆)-alkenyl, -CₙH₂ₙ-R(7) or CF₃,
n is zero, 1, 2, 3 or 4,
R(7) is (C₃-C₇)-cycloalkyl or phenyl,
which is unsubstituted or substituted by 1-3 substituents from the group comprising F, Cl, CF₃, methyl, methoxy and NR(8)R(9);
R(8) and R(9) are H or (C₁-C₄)-alkyl, where R(5) also has the meaning of H,
R(6) is H or (C₁-C₄)-alkyl,
where R(5) and R(6) together can be 4 or 5 methylene groups, of which one CH₂ group can be replaced by oxygen, S, NH, N-CH₃ or N-benzyl,
R(2) is -SR(10), -OR(10), -NHR(10), -NR(10)R(11), -CHR(10)R(12), -CR(12)R(13)OR(13'), -CR(12)[CH₂O-R(13')]R(13), -[CR(17)R(18)]ₚ-CO-[CR(19)R(20)]_{q}-R(14),
R(10) and R(11) are identical or different -[CHR(16)]ₛ-(CH₂)ₚ-(CHOH)_{q}-(CH₂)ᵣ-(CHOH)ₜ-R(21), -(CH₂)ₚ-O-(CH₂-CH₂O)_{q}-R(21),
R(21)
is hydrogen or methyl,
p, q and r are identical or different zero, 1, 2, 3 or 4,
s is zero or 1,
t is 1, 2, 3 or 4,
R(12) and R(13) are identical or different hydrogen, (C₁-C₆)-alkyl or, together with the carbon atom carrying them, are a (C₃-C₈)-cycloalkyl,
R(13') is hydrogen or (C₁-C₄)-alkyl,
R(14) is H, (C₁-C₆)-alkyl, (C₃-C₈)-cylcoalkyl or -CₐH₂ₐ-R(15),
a is zero, 1, 2, 3 or 4,
R(15)
is phenyl,
which is unsubstituted or substituted by 1 - 3 substituents from the group comprising F, Cl, CF₃, methyl, methoxy and NR(8)R(9) where R(8) and R(9) are H or (C₁-C₄)-alkyl,
(C₁-C₉)-heteroaryl,
which is unsubstituted or substituted as phenyl,
(C₁-C₆)-alkyl, which is unsubstituted or substituted by 1 - 3 OH,
R(16), R(17), R(18), R(19) and R(20)
are hydrogen or (C₁-C₃)-alkyl,
R(3) is defined as R(1),
or
is (C₁-C₆)-alkyl or -X-R(22),
X is oxygen, S or NR(16),
R(16)
is H, (C₁-C₃)-alkyl,
where R(22) and R(16) together can also be 4 or 5 methylene groups and one CH₂ group can be replaced by oxygen, S, NH, N-CH₃ or N-benzyl,
R(22)
is defined as R(14);
or its pharmaceutically tolerable salts.

2. A compound of the formula I as claimed in claim 1, in which:
R(1) is hydrogen, F, Cl, -C≡N, -CF₃, R(4)-SOₘ or R(5)R(6)N-SO₂-,
m is zero, 1 or 2,
R(4) and R(5)
are (C₁-C₈)-alkyl, (C₃-C₄)-alkenyl, -CₙH₂ₙ-R(7) or -CF₃,
n is zero or 1,
R(7) is (C₃-C₆)-cycloalkyl or phenyl, which is unsubstituted or substituted by 1 - 3 substituents from the group comprising F, Cl, CF₃, methyl, methoxy and NR(8)R(9) where R(8) and R(9) are H or methyl,
where R(5) also has the meaning of H, R(6) is H or methyl,
R(3) is hydrogen, methyl, cyano, -CF₃, F or Cl, and the other radicals are as defined in claim 1.

3. A compound of the formula I as claimed in claim 1, in which:
R(1) is F, Cl, -C≡N, -CF₃, R(4)-SOₘ or R(5)R(6)N-SO₂-, m is zero, 1 or 2,
R(4) is methyl or CF₃,
R(5) and R(6)
independently of one another are H or methyl;
R(2) is -SR(10), -OR(10), NHR(10), -NR(10)R(11), -CHR(10)R(12), -CR(12)R(13)OR(13'), -CR(12)[CH₂-OR(13')]R(13), -CO-CH₂-R(14) or -CH₂-CO-R(14),
R(10) and R(11) identical or different are -CH₂-(CHOH)_{q}-CHOH-CHOH-CHOH-CH₂OH, -CH₂-CHOH-CH₂OH, -[CHR(16)]ₛ-CH₂-CHOH-R(21) or -(CH₂)ₚ-O-(CH₂-CH₂-O)_{q}-CH₃,
p is zero, 1 or 2,
q is zero, 1 or 2,
s is zero or 1,
R(21)
is hydrogen or methyl,
R(12) and R(13)
are identical or different hydrogen, methyl or, together with the carbon atom carrying them, a (C₃-C₈)-cycloalkyl,
R(13')
is hydrogen or methyl,
R(14) is H, (C₁-C₆)-alkyl, (C₃-C₈)-cycloalkyl, -CₐH₂ₐ-R(15),
a is 0 or 1,
R(15)
is phenyl,
which is unsubstituted or substituted by 1 - 2 radicals from the series comprising F, Cl, CF₃ and -CH₃,
or heteroaryl from the series comprising furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl and pyridazinyl,
which are unsubstituted or substituted by a radical from the series comprising F, Cl, CF₃ and -CH₃, (C₁-C₄)-alkyl which is substituted by an OH;
R(16)
is hydrogen or methyl,
R(3) is methyl, cyano, trifluoromethyl, F, Cl or hydrogen.

4. A compound of the formula I as claimed in claim 1, wherein:
R(15)
is phenyl,
which is unsubstituted or substituted by 1 - 2 radicals from the series comprising F, Cl and CF₃,
imidazolyl, tetrazolyl, pyridinyl or pyrimidinyl,
which are unsubstituted or substituted by a radical from the series comprising F, Cl, CF₃ and CH₃.

5. A process for the preparation of a compound I as claimed in claim 1, which comprises reacting a compound of the formula II 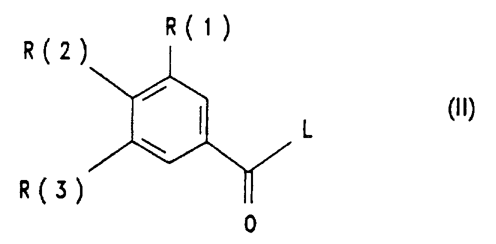 with guanidine, in which R(1) to R(3) have the given meaning and L is a leaving group which can be easily nucleophilically substituted.

6. The use of a compound I as claimed in claim 1 for the production of a medicament for the treatment of arrhythmias.

7. The use of a compound I as claimed in claim 1 for the production of a medicament for the treatment or prophylaxis of cardiac infarct.

8. The use of a compound I as claimed in claim 1 for the production of a medicament for the treatment or prophylaxis of angina pectoris.

9. The use of a compound I as claimed in claim 1 for the production of a medicament for the treatment or prophylaxis of ischemic conditions of the heart.

10. A medicine comprising an effective amount of a compound I as claimed in one or more of claims 1 to 4.

## Revendications

1. Composé de formule I 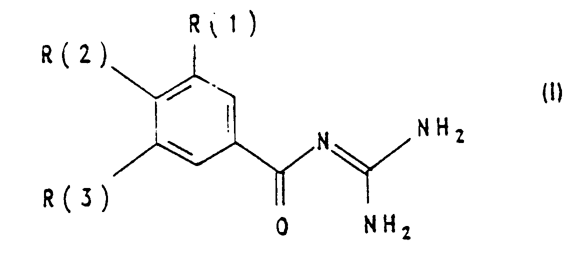 dans laquelle
R(1) représente un atome d'hydrogène ou de F, Cl, Br, I, ou un groupe -NO₂, -C≡N, -CF₃, R(4)-SOₘ ou R(5)R(6)N-SO₂-,
m est zéro, 1 ou 2,
R(4) et R(5) représentent un groupe alkyle en C₁-C₈, alcényle en C₃-C₆, -CₙH₂ₙ-R(7), CF₃,
n est zéro, 1, 2, 3 ou 4,
R(7) représente un groupe cycloalkyle en C₃-C₇, phényle qui est non substitué ou substitué par 1-3 substituants choisis parmi F, Cl, CF₃, le groupe méthyle, méthoxy ou NR(8)R(9), R(8) et R(9) représentant H ou un groupe alkyle en C₁-C₄,
R(5) représentant également H,
R(6) représente H ou un groupe alkyle en C₁-C₄,
R(5) et R(6) pouvant être ensemble 4 ou 5 groupes méthylène, dont un groupe CH₂ peut être remplacé par un atome d'oxygène ou de S ou par un groupe NH, N-CH₃ ou N-benzyle,
R(2) représente -SR(10), -OR(10, -NHR(10), -NR(10)R(11), -CHR(10)R(12), -CR(12)R(13)OR(13'), -CR(12)[CH₂O-R(13')]R(13), -[CR(17)R(18)]ₚ-CO-[CR(19)R(20)]_{q}-R(14), R(10), R(11) sont identiques ou différents et représentent
-[CHR(16)]ₛ-(CH₂)ₚ-(CHOH)_{q}-(CH₂)ᵣ-(CHOH)ₜ-R(21) -(CH₂)ₚ-O-(CH₂-CH₂O)ᵣ-R(21),
R(21) représente un atome d'hydrogène ou le groupe méthyle,
p, q, r sont identiques ou différents et représentent zéro, 1, 2, 3 ou 4,
s est zéro ou 1,
t est 1, 2, 3 ou 4,
R(12), R(13) sont identiques ou différents et représentent un atome d'hydrogène ou un groupe alkyle en C₁-C₆ ou, conjointement avec l'atome de carbone qui les porte, un groupe cycloalkyle en C₃-C₈,
R(13') représente un atome d'hydrogène ou un groupe alkyle en C₁-C₄,
R(14) représente H ou un groupe alkyle en C₁-C₆, cycloalkyle en C₃-C₈, -CₐH₂ₐ-R(15), a est zéro, 1, 2, 3 ou 4,
R(15) représente un groupe phényle qui est non substitué ou substitué par 1 à 3 substituants choisis parmi F, Cl, CF₃, le groupe méthyle, méthoxy ou NR(8) R(9),
R(8) et R(9)
représentant H ou un groupe alkyle en C₁-C₄,
un groupe hétéroaryle en C₁-C₉ qui est non substitué ou substitué comme le groupe phényle,
un groupe alkyle en C₁-C₆ qui est non substitué ou substitué par 1-3 groupes OH,
R(16), R(17), R(18), R(19), R(20) représentent un atome d'hydrogène ou un groupe alkyle en C₁-C₃,
R(3) est défini comme R(1),
ou
représente un groupe alkyle en C₁-C₆ ou -X-R(22),
X représente un atome d'hydrogène ou de S ou un groupe NR(16),
R(16) représente un atome d'hydrogène ou un groupe alkyle en C₁-C₃,
R(22) et R(16) pouvant également représenter ensemble 4 ou 5 groupes méthylène, et un groupe CH₂ pouvant être remplacé par un atome d'oxygène ou de S ou par un groupe NH, N-CH₃ ou N-benzyle,
R(22) est défini comme R(14);
et ses sels pharmaceutiquement acceptables.

2. Composé de formule I selon la revendication 1, dans lequel
R(1) représente un atome d'hydrogène ou de F, Cl, -C≡N, -CF₃, R(4)-SOₘ ou R(5)R(6)N-SO₂-,
m est zéro, 1, 2,
R(4) et R(5) représentent un groupe alkyle en C₁-C₈, alcényle en C₃-C₄, -CₙH₂ₙ-R(7), -CF₃,
n est zéro ou 1,
R(7) représente un groupe cycloalkyle en C₃-C₆, phényle
qui est non substitué ou substitué par 1-3 substituants choisis parmi F, Cl, CF₃, le groupe méthyle, méthoxy ou NR(8)R(9),
R(8) et R(9)
représentant H ou le groupe méthyl,
R(5) ayant également la signification de H,
R(6) est H ou le groupe méthyle,
R(3) représente un atome d'hydrogène ou de F, Cl ou le groupe méthyle, cyano ou CF₃,
et les autres radicaux sont tels que définis dans la revendication 1.

3. Composé de formule I selon la revendication 1 dans lequel
R(1) représente F, Cl, -C≡N, -CF₃, R(4)-SOₘ ou R(5)R(6)N-SO₂-,
m est zéro, 1, 2,
R(4) représente le groupe méthyle ou CF₃,
R(5) et R(6) représentent, indépendamment l'un de l'autre, H ou le groupe méthyle;
R(2) = -SR(10), -OR(10), NHR(10), -NR(10)R(11), -CHR(10)R(12), -CR(12)R(13)OR(13'), -CR(12)[CH₂O-R(13')]R(13), -CO-CH₂-R(14) ou -CH₂-CO-R(14),
R(10), R(11) étant identiques ou différents et représentant -CH₂-(CHOH)_{q}-CHOH-CHOH-CHOH-CH₂OH, -CH₂-CHOH-CH₂OH, -[CHR(16)]ₛ-CH₂-CHOH-R(21) ou -(CH₂)ₚ-O-(CH₂-CH₂-O)_{q}-CH₃,
p est zéro, 1, 2,
q est zéro, 1, 2,
s est zéro, 1,
R(21) représente un atome d'hydrogène ou le groupe méthyle,
R(12), R(13) sont identiques ou différents et représentent un atome d'hydrogène ou le groupe méthyle ou, conjointement avec l'atome de carbone qui les porte, un groupe cycloalkyle en C₃-C₈,
R(13') représente un atome d'hydrogène ou le groupe méthyle,
R(14) représente H ou un groupe alkyle en C₁-C₆, cycloalkyle en C₃-C₈, -CₐH₂ₐ-R(15),
a est zéro ou 1,
R(15) représente un radical phényle qui est non substitué ou porte 1-2 substituants choisis parmi F, Cl, CF₃, -CH₃,
ou un radical hétéroaryle choisi parmi les groupes furannyle, thiényle, pyrrolyle,imidazolyle, pyrazolyle, triazolyle, tétrazolyle, oxazolyle, isoxazolyle, thiazolyle, isothiazolyle, pyridyle, pyrazinyle, pyrimidinyle, pyridazinyle,
qui est non substitué ou porte un substituant choisi parmi F, Cl, CF₃, -CH₃,
un radical alkyle en C₁-C₄ qui est substitué par un groupe OH;
R(16) représente un atome d'hydrogène ou le groupe méthyle,
R(3) représente un atome d'hydrogène ou de F, Cl ou le groupe méthyle, cyano ou trifluorométhyle.

4. Composé de formule I selon la revendication 1, caractérisé en ce que
R(15) représente un radical phényle
qui est non substitué ou porte 1-2 substituants choisis parmi F, Cl, CF₃, -CH₃,
un radical imidazolyle, tétrazolyle, pyridinyle, pyriidinyle
qui sont non substitués ou portent un substituant choisi parmi F, Cl, CF₃, CH₃.

5. Procédé pour la préparation d'un composé I selon la revendication 1, caractérisé en ce que l'on fait réagir avec de la guanidine un composé de formule II 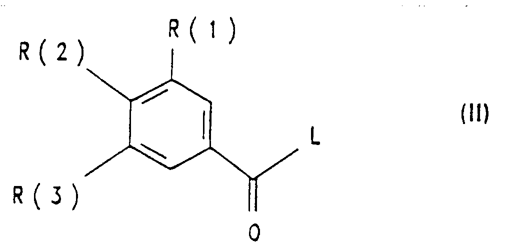 dans laquelle R(1) à R(3) ont les significations indiquées et L représente un groupe partant à substitution nucléophile aisée.

6. Utilisation d'un composé I selon la revendication 1, pour la fabrication d'un médicament destiné au traitement d'arythmies.

7. Utilisation d'un composé I selon la revendication 1, pour la fabrication d'un médicament destiné au traitement ou à la prophylaxie de l'infarctus du myocarde.

8. Utilisation d'un composé I selon la revendication 1, pour la fabrication d'un médicament destiné au traitement ou à la prophylaxie de l'angine de poitrine.

9. Utilisation d'un composé I selon la revendication 1, pour la fabrication d'un médicament destiné au traitement ou à la prophylaxie d'états ischémiques du coeur.

10. Médicament contenant une quantité efficace d'un composé I selon une ou plusieurs des revendications 1 à 4.
